# EUROPEAN PATENT APPLICATION

(11) **EP 1 598 080 A1**
(43) Date of publication of application: **23.11.2005**
(21) Application number: 04712751.9
(22) Date of filing: 19.02.2004
(51) Int. Cl.: A61K 47/10, A61K 47/26, A61K 9/48

(54) **SEAMLESS CAPSULE**

(30) Priority: 20.02.2003 JP 2003042744
(71) Applicant: Freund Corporation, Tokyo 163-6034 (JP)
(72) Inventor: SUZUKI, Katsuhiro, c/o Freund Industrial Co., Ltd., Hamamatsu-shi, Shizuoka 431-2103 (JP)
(74) Representative: Eisenführ, Speiser & Partner
(86) International application number: PCT/JP2004/001928
(87) International publication number: WO 2004/073744

(57) **Abstract**

A seamless capsule including a filler material and a shell which encapsulates the filler. The shell comprises (a) a shell material and (b) a crystallization agent which is one or two or more selected from a group consisting of sorbitol, mannitol, xylitol, erythritol, paratinitt, lactitol, maltitol, trehalose, and saccharose. The shell includes crystals which are formed by deposition of the crystallization agent when a shell liquid including the shell material and the crystallization agent is hardened, and the shell is substantially opaque due to the presence of the crystal.

## Description

### TECHNICAL FIELD

The present invention relates to a seamless capsule in which a filler material such as a food, health food, pharmaceutical, flavoring, or condiment, is encapsulated by a shell material such as gelatin or agar, and a manufacturing method thereof. In particular, the present invention relates to a seamless capsule wherein light shielding ability and anti-stickiness ability of a shell of the capsule are improved.

Priority is claimed on Japanese Patent Application No. 2003-42744, filed February 20, 2003, the content of which is incorporated herein by reference.

### BACKGROUND OF THE INVENTION

Conventionally, a technology for manufacturing capsules without seams in the shell, that is, a seamless capsule, has been known. In particular, as a technology suitable for manufacturing a capsule being smaller than a typical soft capsule and larger than a microcapsule, methods and apparatuses for producing seamless capsules enclosing a liquid inner layer have been proposed in which multilayer liquid drops are formed by extruding a multilayer liquid jet from a multiple nozzle such as a double nozzle, triple nozzle, or the like, and the outermost liquid layer of the multilayer liquid drop is hardened by bringing it into contact with a hardening liquid to form a shell.

For example, please refer to Patent documents 1 to 16.
Patent document 1: Japanese Unexamined Patent Application, First Publication No. Sho 59-11859
Patent document 2: Japanese Unexamined Patent Application, First Publication No. Sho 62-176536
Patent document 3: Japanese Unexamined Patent Application, First Publication No. Sho 62-180744
Patent document 4: Japanese Unexamined Patent Application, First Publication No. Hei 4-322740
Patent document 5: Japanese Unexamined Patent Application, First Publication No. Hei 4-322741
Patent document 6: Japanese Unexamined Patent Application, First Publication No. Hei 5-228360
Patent document 7: Japanese Unexamined Patent Application, First Publication No. Hei 4-338230
Patent document 8: Japanese Unexamined Patent Application, First Publication No. Hei 5-200274
Patent document 9: Japanese Unexamined Patent Application, First Publication No. Hei 5-200275
Patent document 10: Japanese Unexamined Patent Application, First Publication No. Hei 5-200276
Patent document 11: Japanese Unexamined Patent Application, First Publication No. Hei 5-138012
Patent document 12: Japanese Unexamined Patent Application, First Publication No. Hei 6-134292
Patent document 13: Japanese Unexamined Patent Application, First Publication No. Hei 6-154587
Patent document 14: Japanese Unexamined Patent Application, First Publication No. Hei 8-10313
Patent document 15: Japanese Unexamined Patent Application, First Publication No. Hei 8-26976
Patent document 16: Japanese Unexamined Patent Application, First Publication No. Hei 9-155183

However, the capsule surface of such a type of seamless capsules or soft capsule tends to be sticky, and therefore there is a problem in which the capsules adhere to each other when they are used. Conventionally, techniques for improving the anti-sticking ability conducted by preventing stickiness of the surface of a capsule have been proposed.

For example, please refer to patent documents 17 to 22.
Patent document 17: Japanese Unexamined Patent Application, First Publication No.2001-57848
Patent document 18: Japanese Unexamined Patent Application, First Publication No. Sho 56-156212
Patent document 19: Japanese Unexamined Patent Application, First Publication No. Hei 2-22221
Patent document 20: Japanese Unexamined Patent Application, First Publication No. Hei 10-80466
Patent document 21: Japanese Unexamined Patent Application, First Publication No. Hei 10-310519
Patent document 22: Japanese Unexamined Patent Application, First Publication No. 2001-178376

Furthermore, techniques for improving solubility and anti-insolubilization of a shell are disclosed in which a succinic acid gelatin wherein an amino acid is modified with an organic acid is used. For example, please refer to patent documents 23.
Patent document 23: Japanese Unexamined Patent Application, First Publication No. 2000-44465

However, it is difficult to achieve the prevention of stickiness of seamless capsules by using the techniques of patent documents 17 to 23. That is, there are the following problems.
Patent document 17 discloses a technique for preventing moisture absorption. The technique has a problem in that since the production method of the technique includes a step of applying heat, a capsule may be dissolved by the heat and commercial value thereof is lost when the technique is applied to seamless capsules.
Patent document 18 discloses a technique for treating a surface of a capsule with carnauba wax. In the technique, although a capsule having a moisture content of about 5 to 10% is treated, treatment of capsules having a moisture content of 10% or more is not mentioned.
Patent document 19 discloses a technique wherein natural calcium is compounded in a shell. However, there is a problem in that stability of the seamless capsules cannot be maintained when the seamless capsules are formed.
Patent documents 20 and 21 disclose techniques wherein a treated milk protein or flour is compounded in a shell. However, the use of the techniques is limited to a soft capsule. Therefore, although some effects may be obtained to some extent, the problem of stickiness of capsules has not been solved.
Patent document 22 mentions a technique wherein an anti-sticking layer is formed by providing a powder on the surface of a capsule. However, this technique is has a problem in that this technique is limited to a seamless soft capsule, and production cost is increased since the technique is required to conduct a further three steps of applying a powder, removing a powder, and removing fat and oil used.
Patent document 23 does not mention capsules comprising gelatin and/or agar.

As described above, the prior art has problems such as the techniques not applicable to seamless capsules, stickiness preventing ability being insufficient, production cost being increased, and the like. A technique wherein a problem of adhesion between seamless capsules can be overcome at low cost has not been developed yet.

Since a shell including a shell material such as gelatin and agar, which has been used for forming seamless capsules, has high stickiness under the condition of high temperature and high humidity, these is a possibility that problems are caused such as seamless capsules being adhered to each other to form an aggregate and thereby it becoming difficult to take the capsules out from a vessel or the like, a shell being broken when a capsule is removed from the aggregate of capsules, or the like. Furthermore, since a shell of a seamless capsule has high stickiness, depending on the environmental conditions, production efficiency may be lowered since fliction lessness of seamless capsules may deteriorate, which ability is required in a step of bottling seamless capsules formed, a step of packaging seamless capsules by PTP (press through package) and the like. In this way, stickiness preventing methods are a very important subject in the field of the seamless capsules.

Furthermore, recently, in addition to a technique of adding a plasticizer such as glycerin to a shell, the use of seamless capsules wherein seamless capsules having an oral quick-dissolving property of easily breaking down in the oral cavity have been noted in the fields of foods and pharmacy. The oral quick-dissolving seamless capsules have a shell which is soft and tends to be broken. Accordingly, it is required to provide a further improved a function of preventing stickiness, and a technique therefore is anxiously required to be provided.

Furthermore, another problem of seamless capsules to be overcome is to obtain a light shielding type shell without using a colorant or inorganic powder. Such a shell has been difficult to produce.

A shell which is transparent and colorless is generally used as a shell of a seamless capsule, and a colored shell also can be used if required. However, in the pharmacy field and the like, it is necessary to use a light shielding type shell when the material which does not have optical stability is encapsulated in a capsule.
Conventionally, in order to obtain a light shielding type shell, a light shielding agent, in general, inorganic powder such as titanium dioxide, is added to a shell, in order to increase light shielding effects. However, when such a kind of inorganic powder is added to the shell, formability of the capsule may deteriorate, and hardness, flexibility, and stability of the capsule may deteriorate, and/or breakage and deformation of capsules may be caused at the time of the capsule formation. Furthermore, when inorganic powder is added to a shell, influence on taste caused by the inorganic powder is high, and therefore, addition of the inorganic powder may be undesirable in some cases.

An object of the present invention is to provide a seamless capsule having excellent anti-stickiness ability and light shielding ability, and which does not adhere to other capsules or to a vessel, and which does not substantially adhere to a tooth when it is chewed.

### DISCLOSURE OF INVENTION

A seamless capsule of the present invention is a capsule comprising a filler material and a shell which encapsulates the filler, wherein the shell includes (a) a shell material and (b) a crystallization agent which is at least one or two or more selected from a group consisting of sorbitol, mannitol, xylitol, erythritol, paratinitt, lactitol, maltitol, trehalose, and saccharose. The shell includes crystals which are formed by deposition of the crystallization agent when a shell liquid including the shell material and the crystallization agent is hardened. The shell is substantially non-transparent (opaque) due to the presence of the crystals.

The shell may comprise at least one or two or more plasticizers selected from a group consisting of (c) glycerol, propylene glycol, and polyethylene glycol.

The amount of the aforementioned crystallization agent added may be 10 to 80% by mass based on the total amount of the shell, excluding water.

The mass ratio of the aforementioned shell to a filler material (mass of a shell: mass of a filler material) may be 5:95 to 70:30.

### BRIEF DESCRIPTION OF THE DRAWINGS

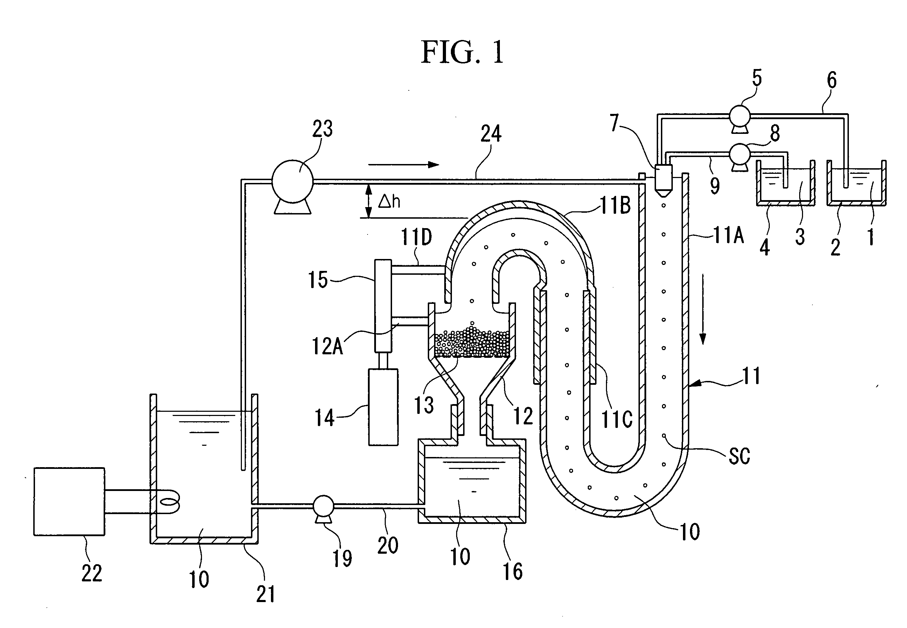

Fig. 1 is a schematic view of an in-liquid nozzle type seamless capsule manufacturing apparatus that can be used for forming a seamless capsule of the present invention.

### Brief Description of the Reference Symbols

1: Core liquid
3: Shell liquid
7: Multiple nozzle
10: Hardening liquid
11: Flow duct
12: Separator
13: Mesh
14: Drive source
SC: Seamless capsule

### BEST MODE FOR CARRYING OUT THE INVENTION

Below, embodiments of the present invention will be explained in detail, referring to the figure. However, the present invention is not limited by following embodiments, and for example, the constituents of the embodiments may be combined with each other appropriately.

A seamless capsule of the present invention includes a filler material and a shell which encapsulates the filler material. The shell comprises (a) a shell material and (b) a crystallization agent, and if required, (c) plasticizer. The shell includes crystals which are formed by deposition of a crystallization agent when a shell solution including a shell material and a crystallization agent is hardened. The shell is substantially opaque due to the presence of the crystal. The (c) component is an optional component and may be not included in the shell. General seamless capsules include a plasticizer in the shell, and when the present invention is applied to oral quick-dissolving seamless capsules whose shell breaks easily in the oral cavity, the (c) component can be included as an essential component.

As the (a) shell material which is an essential component of the seamless capsule of the present invention, one of or two or more of organic polymers for forming a shell such as gelatin, casein, zein, pectin and derivatives thereof, alginic acid and its salts, agar, tragacanth gum, guar gum, locust bean gum, carragheenan, tamarind, mannan, hemilose, starch, chitosan, and the like, which are permitted in terms of food production or pharmacology, can be used. Preferably, gelatin or agar is used alone as the (a) shell material, or a mixed material wherein the gelatin or agar as a main component and one or more other shell materials are comprised is used as the (a) shell material.

As the (b) crystallization agent, which is an essential component of the seamless capsule of the present invention, one or two or more compounds selected from the group consisting of sorbitol, mannitol, xylitol, erythritol, paratinitt, lactitol, maltitol, trehalose, and saccharose can be used. Crystalline sugar alcohol such as sorbitol, mannitol, and the like is preferable in particular.

The addition amount of the crystallization agent is preferably 10 to 80% by mass, and more preferably 10 to 50% by mass, based on the total amount of the shell, wherein amount of water is excluded from the total amount. When the amount of the crystallization agent is less than the aforementioned range, crystals are not formed in the hardened shell, or only few crystals are formed. Accordingly, anti-sticking effects and light shielding effects obtained are insufficient. On the other hand, if the amount of the crystallization agent exceeds the aforementioned range, the hardness and stability of the shell formed may get worse, and deformation and collapse of capsules may tend to be caused when capsules are formed.

As the (c) plasticizer which can be added to the shell, one or more plasticizer selected from a group consisting of glycerin, propylene glycol, polyethylene glycol can be used as the plasticizer, and among them, glycerin is suitable as a plasticizer. The added amount of this plasticizer is 0 to 70% by mass with respect to the total amount of the shell material (the total amount of the components of the shell liquid excluding water). When the (c) plasticizer is comprised in oral quick-dissolving seamless capsules, the added amount of the plasticizer is preferably 15 to 65% by mass, and more preferably 20 to 60% by mass. By mixing glycerin in the shell material within these ranges, the shell becomes pliable after hardening, and dissolves easily in the oral cavity.

In addition to the aforementioned components (a) to (c), the shell can comprise additives which are permitted in terms of food production or pharmacology. For example, by adding a thickening polysaccharide, a gelling agent, a proteolytic agent or the like, it is possible to improve the long-term stability of the shell. The shell can be colored to any arbitrary color tone by a colorant, and flavorings, sweeteners, souring agents or the like can be added to the shell. The thickening polysaccharides, gelling agents, proteolytic agents and the like are added at 10% by mass or less with respect to the total amount of the shell material, and preferably at 5% by mass or less.

The filler material may contain, in addition to the main component (the effective ingredient in the case of a pharmaceutical) such as a foodstuff, health food, flavoring, condiments, pharmaceutical, aromatic agent, or the like, various additives such as solvents (for example, edible oils), sweeteners, souring agents, flavorings, colorings, thickeners (gelatinizing agents), stabilizers, and emulsifiers, or the like that are permitted in terms of food production or pharmacology. When the filler material is prepared in a liquid state, it can take the form of a transparent solution, suspension, orlatex (cream) where the main component is dissolved in a solvent.

The method in which a liquid filler material, that is, a core liquid, is prepared can be any well-known method in the fields of food production or pharmaceutical manufacturing. For example, to prepare a transparent core liquid, the main component and additives are measured and mixed with a solvent such as an edible oil, and as needed heated and agitated to produce a uniform solution.

To prepare an emulsified core liquid, well-known conventional methods can be used in which the main component, including an emulsifying agent, and an oil component are emulsified using a homogenizer to obtain an oil-in-water emulsion. Materials such as super-sweet sweeteners, for example, aspartame or sucralose, that can be dispersed and dissolved in ethanol have large particle diameters when dispersed directly in oil, which causes the capsule formation properties to become unstable. Thus, a method can be used wherein first these are dispersed and dissolved in ethanol using a homogenizer, and then dissolved in oil.

A mass ratio of the shell material to the filler material is preferably 5:95 to 70:30, and more preferably 10:90 to 40:60. When the amount of the shell is less than this range, thickness of the shell is too low, and stable formation of capsules becomes difficult. On the other hand, when the amount of shell exceeds this range, the shell is difficult to break and to dissolve, and an amount of the filler material which can be comprised in one capsule becomes small, and therefore, this is unpreferable from viewpoint of functions as a capsule.

The shell comprises crystals formed by deposition of a crystallization agent, which are deposited when a shell solution including a shell material and a crystallization agent is hardened. The shell is substantially non-transparent due to the presence of the crystals. The shell is substantially opaque due to the presence of the crystals. Since the crystals are exposed on the surface of the shell, stickiness of the shell is not sensed, adhesion between capsules is not caused, and anti-adherence ability for preventing adherence of the capsule to teeth at the time of chewing is sufficient.

The crystals formed in the shell may change according to the kind of crystallization agent used. For example, when crystalline sugar alcohol such as sorbitol and mannitol is used as a crystallization agent, crystals are deposited with a quite uniform grain size and density in the shell, and therefore the shell becomes opaque on appearance, and the surface of the shell has state where fine powders appear to be adhered to the surface.

Due to the effects of the crystals exposed on the surface of the shell, the seamless capsules of the present invention are not adhered to each other, sufficient sticking prevention property with respect to teeth can be achieved at the time of chewing, and capsules contained in a vessel or the like do not form a lump by adhesion between capsules, and therefore capsules can be taken out from the vessel one by one. Furthermore, frictionlessness ability of the seamless capsules is good when they are formed, and formation of seamless capsules is easy since the capsules do not adhere to each other.

The seamless capsules of the present invention have excellent light shielding ability due to the crystal exposed on the surface of the shell and deposited in the shell. Accordingly, even if a substance which does not have optical stability is encapsulated by the shell, unpreferable influence to the taste and the like, which is caused by adding colorant for light shielding or a light shielding agent such as inorganic powder, can be eliminated, since the use of colorant or a light shielding agent is not required in the present invention.

The aforementioned seamless capsule can be manufactured, for example, by conducting the following steps A to E in order.

Step A: preparing the core liquid that includes the filler material and the shell liquid in which a shell material is dissolved.

Step B: using a coaxial multiple nozzle having the inner nozzle and the outer nozzle that surrounds the inner nozzle, supplying the core liquid to the inner nozzle and the shell liquid to the outer nozzle so as to extrude them, and forming multilayer liquid drops by extruding a multilayer jet from the coaxial multiple nozzle.

Step C: hardening the shell liquid while the multilayer liquid drops flow in a hardening liquid that flows through a pass, and forming seamless capsules in which the core liquid is surrounded by the shell material.

Step D: separating the seamless capsules from the hardening liquid that surrounds them.

Step E: eliminating the hardening liquid adhering to the surface of the seamless capsules that have been separated from the hardening liquid, and at the same time forming seamless capsules that do not substantially stick to each other by drying their surfaces.

Herein after, each step is explained in detail.

### Step A

A shell liquid in which a shell material is dissolved is prepared. The shell liquid is prepared by dissolving a shell material in which (a) a shell material and (b) a crystallization agent, are included and, if required, (c) a plasticizer material and/or additives included in a suitable amount of water while heating. The amount of the shell material comprised in the shell liquid (the total amount of the components of the shell liquid excluding water) is about 10 to 50% by mass, based on the total amount of the shell liquid, and more preferably about 20 to 40% by mass. For example, in the case of gelatin, the comprised amount of the shell material is 15 to 35% by mass, based on the total amount of the shell liquid, and preferably 20 to 30% by mass.

The method of adding water to the shell forming agent and dissolving it by heat is not limited. For example, a method in which water is added to the shell forming agent and it is dissolved by heating after swelling, or a method in which the shell forming agent is injected into heated water and dissolved by agitation can be used. In this heated water-agitation dissolving method, the adjustment of the liquid can be done in a short time.
The heating temperature is set according to the type of shell forming agent used, and for example, in the case of gelatin, is 45 to 90°C, and preferably, 45 to 55°C. In order to prepare the shell liquid so as not to incorporate bubbles, preferably the shell liquid is prepared while the shell material and water are injected into a heated reduced-pressure tank, and heated and agitated under a reduced pressure atmosphere.

The core liquid and the shell liquid prepared as described above are stored in suitable vessels such as separate storage tanks. The shell liquid must be cooled and maintained at a temperature that does not allow gelling. The storage temperature of the shell liquid is set depending on the type of shell forming agent that is used, and for example, in the case of gelatin, is 45 to 90°C, and preferably 45 to 55°C. With regards to the prepared amounts of the core liquid and the shell liquid, in the completed seamless capsules, preferably the mass ratio of the shell material and the filler material is set between 5:95 to 70:30.

### Step B to step D

Steps B to D can be carried out sequentially by using a conventionally well-known seamless capsule manufacturing apparatus. Fig. 1 is a schematic drawing showing an example of a suitable manufacturing apparatus for continuously carrying out steps B to D of the manufacturing method.

In the in-liquid nozzle type seamless capsule manufacturing apparatus in Fig. 1, the core liquid (the inner layer liquid) 1 for forming the seamless capsules is stored in the core liquid tank 2, and the shell liquid (the outer layer liquid) 3 for covering the core liquid 1 is stored in the shell liquid tank 4.

The core liquid 1 is delivered under pressure to the multiple nozzle 7 via the duct 6 from the core liquid tank 2 by the pump 5, and the shell liquid 3 is delivered under pressure to the multiple nozzle 7 via the duct 9 from the shell liquid tank 4 by the pump 8.

The multiple nozzle 7 is formed so as to be inserted into the opening of the flow duct 11, that is, the inflow portion 11A of the hardening liquid 10, and generate multilayer liquid drops by extruding the core liquid 1 and the shell liquid 3 into the hardening liquid 10 in the flow duct 11.

The hardening liquid 10 cools and hardens the generated multilayer liquid drops to form the seamless capsules SC. When the shell liquid is hardened by cooling, edible oils such as medium chain triglyceride (MCT) can be used as a hardening liquid, or a hardening liquid can be used whose property of preventing sticking between multilayer liquid drops is improved by adding a surfactant such as lecithin to this MCT. The temperature of the hardening liquid is set from 0°C to room temperature.

In the present apparatus, the flow duct 11 is formed as a curved cylinder consisting of a substantially J-shaped inflow part 11A and an inverted J-shaped outflow part 11B that engages the inflow part 11A by being inserted therein so as to be able to slide while forming an air-tight seal by the engagement part 11C. Therefore, the flow duct 11 is formed so that the inflow part 11A and the outflow part 11B can move relatively to each other at the engagement part 11C. In particular, the present apparatus is structured so that the outflow part 11B moves vertically. Thereby, the difference Δh between the heights of the liquid surface of the inflow part 11A and the liquid surface of the outflow part 11B of the flow duct 11 can be adjusted optionally by moving the outflow part 11B vertically between the rise position and the down position thereof. By adjusting Δh, which is a difference between the heights of the liquid surfaces, the flow rate of the hardening liquid 10 in the flow duct 11 can be adjusted.

A substantially funnel-shaped separator 12 is disposed below the outlet end of the outflow part 11B of the flow duct 11. This separator 12 separates the seamless capsules SC and the hardening liquid 10 that flow out together from the flow duct 11. Inside the separator 12, a mesh 13 is stretched out through which only the hardening liquid 10 passes, not the seamless capsules SC.

The separator 12 moves vertically with the outflow part 11B of the flow duct 11 by, for example, a pressure flow cylinder such as an air cylinder or hydraulic cylinder, or a motor. Specifically, a part of the outflow part 11B of the flow duct 11 is joined to a connecting rod 11D while the separator 12 is joined to the connecting rod 12A. Furthermore, the connecting rods 11D and 12A are joined to a connecting member 15, and this connecting member 15 is joined to a part of the drive source 14, such as the piston rod of an air cylinder.

Therefore, when the drive source 14 is activated, and, for example, the piston rod of an air cylinder moves reciprocally in the vertical direction, the outflow part 11 B of the flow duct 11 and the separator 12 move vertically together equal distances due to the connecting member 15 and each of the connecting rods 11D and 12A. Accordingly, a constant difference is maintained between the height of the liquid surface of the hardening liquid 10 in the outflow part 11B and the separator 12 (in particular, the mesh 13 thereof).

In the separator 12, the hardening liquid 10 separated from the seamless capsules SC is recovered in the separation tank 16 therebelow.

The small diameter part at the bottom end of the separator 12 engages with the cylinder part of the upper end of the separation tank 16, and the separator 12 is structured so that even if it moves vertically, it does not separate from the separation tank 16.

The hardening liquid 10 inside the separation tank 16 is delivered under pressure to the cooling tank 21 via the duct 20 by the pump 19. The hardening liquid 10 inside the cooling tank 21 is cooled to a predetermined temperature, and then is returned into the flow duct 11 via the duct 24.

This seamless capsule manufacturing apparatus has an in-liquid nozzle structure, and thus the multiple nozzle 7 is structured such that it is inserted into the entrance part of the flow duct 11 that forms the flow path for supplying the hardening liquid 10, the core liquid 1 and the shell liquid 3 are extruded into the liquid, and the latter encapsulates the former completely.

Therefore, in the present embodiment, the core liquid 1 and the shell liquid 3 that are extruded from the multiple nozzle 7 are formed into multilayer liquid drops in the hardening liquid 10 in the flow duct 11 (step B), and they are cooled and hardened by the action of the hardening liquid 10 as they flow through the flow duct 11 (step C). Next, the seamless capsules SC formed in this manner flow down along with the hardening liquid 10 onto the mesh 13 of the separator 12 from the exit end of the outflow part 11B of the flow duct 11, and are separated from the hardening liquid 10 by the mesh 13 (step D). The hardening liquid 10 passes through the mesh 13 to be recovered in the separation tank 16. The seamless capsules SC that have accumulated on the mesh 13 are later recovered in a product recovery vessel (not illustrated) in batch when an appropriate amount has been accumulated.

In the present apparatus, flow rate of the hardening liquid 10 which flow in the flow duct 11 can be controlled, and a constant difference can be maintained between the liquid surfaces of the hardening liquid 10 in the outflow part 11B and the separator 12. Therefore, the seamless capsules SC in the present embodiment always have the desired spherical shape, and furthermore, breakage and leaking of the seamless capsules SC can be prevented. In the present invention, the particle diameter of the seamless capsules SC is 0.5 to 20mm, preferably 1 to 15mm, and more preferably 1 to 10mm. Seamless capsules SC having a particle diameter in this range are easily manufactured, they are easily handled by the user, and they are an appropriate size for oral use.

In step B, in addition to the coaxial double nozzle, a coaxial triple nozzle can also be used, and in addition to the nozzle oscillating method, the oscillating method necessary for generating the multilayer liquid drops includes various methods such as ring oscillation methods and tube oscillation methods as well. Of course, instead of a multiple nozzle, a single nozzle that extrudes a liquid drop having only a single layer can be used.

In step B, preferably a multiple nozzle 7 is used whose distal angle (the angle of the conical part) is equal to or less than 90°. By using a multiple nozzle 7 whose distal angle is equal to or less than 90°, no turbulence occurs in the flow of the hardening liquid that flows in contact with the distal end of the multiple nozzle 7, and seamless capsules SC having uniform particle diameters can be formed.

In step B, the respective distal ends of the outer nozzle and the inner nozzle of the multiple nozzle 7 can be disposed on the same plane, or a structure can be used wherein the distal end of the inner nozzle protrudes out 1 to 5mm from the distal end of the outer nozzle.

In step B, the position of the distal end of the multiple nozzle 7 can be aligned with the central axis of the flow duct 11, or can be disposed eccentric to the central axis. In the case that the position of the distal end of the multiple nozzle 7 is aligned with the central axis of the flow duct 11, the multilayer liquid drops extruded from the multiple nozzle 7 drop straight down along the central axis of the multiple nozzle 7. In contrast, in the case that the position of the distal end of the multiple nozzle 7 is disposed eccentric to the central axis, the multilayer liquid drops fall along a spiral shaped path in proximity to the inner wall of the flow duct 11, and thereby it is possible to lengthen the amount of time of the downward flow of the multilayer liquid drops. By making the position of the multiple nozzle 7 variable with respect to the central axis of the flow duct 11, it is possible to adjust the finished state of the seamless capsules, and it is possible to obtain a high quality product by preventing the occurrence of irregularities in thickness and eyes which are small liquid cells formed in a shell layer.

In steps B and C, a structure is preferable wherein a stroboscope is disposed at a position along the flow duct 11, and thereby the particle diameter and shape of the multilayer liquid drops flowing down through the flow duct 11 can be monitored. By monitoring the multilayer liquid drops using a stroboscope in this manner, it is possible to rapidly adjust the extrusion conditions of each liquid from the multiple nozzle 7, the oscillation conditions, the flow rate of the hardening liquid, and the like so as to make the particle diameter of the seamless capsules to be manufactured, and it is possible to manufacture without waste seamless capsules having the object particle diameter without irregularities in thickness or eyes.

In step C, preferably a structure is used in which the hardening liquid 10 flows in the flow duct 11 through the entire circumference of the upper end of the flow duct 11, and thereby the hardening liquid 10 uniformly flows into the end of the flow duct 11 from all directions. Due to the hardening liquid 10 flowing in through the entire circumference of the end surface of the flow duct 11, it is possible to prevent the occurrence of local turbulence in the hardening liquid flow inside the flow duct 11.

In step C, preferably a dehydration device that eliminates water from the circulating hardening liquid 10 is provided at a location in contact with the hardening liquid 10. There is the possibility that atmospheric water or water from the multilayer liquid drops will become mixed with the hardening liquid 10. When the amount of water in the hardening liquid increases, there is the concern that problems such as the deformation of the shell, the capsules sticking together easily, and variations in the state of hardness of the shell liquid will occur. By providing a dehydration device that decreases as much as possible the amount of water in the hardening liquid 10, it becomes possible to manufacture high quality seamless capsules stably. The dehydration device can be a water absorption type, a cooling trap type, a microwave heating type or the like, and normally a simple device in which the hardening liquid 10 is brought into contact with an absorbent material such as silica gel is used.

In step D, instead the seamless capsules SC flowing down with the hardening liquid 10 onto the mesh 13 of the separator 12 and the seamless capsules SC being separated from the hardening liquid 10 using a mesh 13, a structure can be used wherein a separating and conveying apparatus provided with a conveyor belt made of mesh or a cloth filter is used, the efflux from the flow duct 11 is received by the conveyor belt, the hardening liquid falls through and is recovered, and only the seamless capsules SC are conveyed on the conveyor belt. By using such a separating and conveying apparatus, it is possible to prevent the problem of the separated seamless capsules SC piling up and deforming or crushing the capsules beneath them.

### Step E

Seamless capsules manufactured by steps B through D described above and separated from the hardening liquid have the hardening liquid adhering to the surface thereof removed in step E, and by drying their surface, seamless capsules are formed without substantially sticking to each other. The step E is preferably conducted by the following sub-steps e1 to e7 in order, but does not limit thereto.
Step e1: seamless capsules separated from the hardening liquid in step D are cooled either as they are or by being immersed in a coolant liquid consisting of a fluid that does not dissolve the shell, and specifically maintained between 0°C and 20°C, and preferably between about 1°C and 10°C, and thereby the hardening of the shell is promoted.
Step e2: the cooled seamless capsules are centrifuged, thereby eliminating the liquid adhering to the surface of the capsules;
Step e3: the centrifuged capsules are dried;
Step e4: the dried seamless capsules are cleaned with an organic solvent;
Step e5: the seamless capsules that have been cleaned in an organic solvent are dried;
Step e6: seamless capsules that have completed step e5 are sieved and graded;
Step e7: after drying, sieving, and grading, the seamless capsules are packaged.

In step e1, the cooling method is not particularly limited. It is possible to use, for example, a method in which the seamless capsules that have been separated from the hardening liquid are placed in a tray, a cooling liquid is put therein, each tray is placed in a refrigerator, and they are cooled for a certain period of time; a method in which the seamless capsules are conveyed on a conveyor to be cooled by passing through a tunnel shaped cooler; or a method in which the seamless capsules are brought into contact with a cooling plate. Preferably, a material that does not soften, dissolve, or destroy the shell is used as the cooling liquid. Examples are edible oils such as medium chain triglycerides, or an edible oil that includes a surfactant such as lecithin.

In step e1, by using a cooling temperature of about 2°C, freezing of the water in the capsule shell can be prevented, and at the same time, hardening of the capsule shell can be promoted.

In step e2, the centrifuging conditions are that a liquid such as liquid for hardening, cooling liquid, and oil that adheres to the shell of the seamless capsules is removed in a manner that does not effect the external appearance, and that the revolution speed and time do not deform or break the shell. The cooling liquid on the surface of the seamless capsules is removed as much as possible by this centrifuging, and thereby the drying efficiency in the next drying step e3 is improved, and the time required for drying can be shortened.

In step e2, instead of a process in which oil is removed from the surface of the seamless capsules by centrifuging, the oil on the shell surface can be removed by a process in which the capsules are wiped by a cloth, paper treated so as to become lipophilic, a non-fiber cloth, or the like.

In step e3, the drying method is not particularly limited. Drying can be implemented using methods and apparatuses conventionally used to dry particulate matter. For example, the forced-air drying method (including the fluidized bed drying method), the drum drying method, a reduced pressure drying method, and the like can be used. In the case of the forced-air drying method and the drum drying method, the seamless capsules are brought into contact with an air current equal to or less than the temperature at which the shell softens, preferably 0 to 40°C, and more preferably 10 to 30°C.

In step e3, in the case of using a drum drying method, preferably a baffle is provided that prevents slipping of the seamless capsules inside the drum. When the seamless capsules slip inside the drum, there is the concern that the drying state becomes irregular and thus that seamless capsules that are not completely dried will be produced.

In step e3, in the case of using a continuous-current drying method and the drum drying method, the temperature of the introduced air can be the same from the beginning to the end of the drying (for example, air at room temperature), or the temperature can be varied during the drying. For example, drying can be carried out using an initial cold air equal to or less than 25°C at the start of the drying and then supplying air equal to or greater than 25°C after the passage of a predetermined amount of time. Preferably, the introduced air has a low humidity, and thus as necessary air can be supplied that has been dried by passing through a water absorbing layer such as a silica gel.

In step e3, in the case of using the forced-air or a drum drying method, an aeration plate that carries the seamless capsules and the openings in the drum are preferably selected depending on the particle diameter of the seamless capsules.

Here, for the step e3, a method of immersing a capsule in ethanol and dehydrating as described in Japanese Unexamined Patent Application, First Publication No. Hei 10-211425, or a dry method of contacting an extract which contains ethanol, glycerol, and the like to a capsule and removing moisture described in Japanese Unexamined Patent Application, First Publication No. 2000-126586 can be used. In this case, the following step e4 is skipped.

In step e4, the organic medium used in order to clean the seamless capsules can dissolve the oils (hardening liquid, cooling liquid) that adhere to the seamless capsules. Any organic solvent that does not soften, dissolve, or destroy the shell can be used, and preferably carbohydrates such as ethyl alcohol, ethyl acetate, acetone, hexane, or mixtures thereof are used.

In step e4, the method of cleaning the seamless capsules using the organic solvent is not particularly limited. A method such as immersing the seamless capsules into the organic solvent and lifting them out, a method in which drops or a mist of the organic solvent is dispensed onto the seamless capsules, or the like can be used. The temperature of the organic solvent is about 0 to 40°C, and preferably room temperature. The number of cleanings and the cleaning time are not particularly limited. Cleaning can be carried out one time or repeated a plurality of times. During this cleaning operation, the cleaning efficiency can be improved by agitation or the application of ultrasound to a degree that does not damage the shell of the seamless capsules. The organic solvent is recovered and purified after the cleaning, and reused.

The drying in step e5 (second drying) is carried out mainly to remove the organic solvent from the seamless capsules after cleaning by the organic solvent. This cleaning method is not particularly limited, and can be implemented by using methods and apparatuses conventionally employed to dry particulate matter. For example, the forced-air method (including the fluidized bed drying method), the drum drying method, reduced pressure drying method, centrifuge drying method and the like can be used. The temperature, humidity, and devices are preferably substantially identical to those of the dying step in step e3. The exhaust gas that includes the organic solvent from the drying apparatus undergoes a solvent removal process by being brought into contact with a cooling trap or an appropriate solvent absorbent.

Instead of conducting steps e4 and e5, oils (hardening liquid, cooling liquid) on the surface of the shell may be removed by carrying out rubbing processing of the capsule using cloth, paper on which a lipophilic processing has been carried out, a nonwoven fabric, or the like.

In step e6, the method of sieving and grading the seamless capsules after completion of the second drying step (step e5) can be carried out using methods employed in the product inspection of particulate matter, in particular for encapsulated pharmaceuticals for soft capsules. Inspection categories for the seamless capsules may include the size of the particle diameter, and the presence or absence of abnormally shaped product, broken shell, cloudiness and contaminants, and products with bad external appearance irregularities in thickness, eyes or the like, and fused capsules. With regards to the filler material, various necessary analytic tests in terms of pharmaceutical production and food hygiene are carried out for leaking of the seamless capsules.

In step e7, after completion of the drying and before packaging the seamless capsules after sieving and grading, suitable amounts of a starch such as lactose, mannitol, powdered oblate, corn starch or the like can be sprinkled on the surface of the capsules to prevent sticking, and blocking prevention of the capsules can be implemented

Moreover, the embodiments described above are simply to illustrate examples of the present invention, and the present invention can be modified in various ways without departing from the scope thereof.

### EXAMPLES

Below, the effects of the present invention will be made clear by examples.

### Example 1

Medium chain triglyceride (MCT) was prepared as a filler liquid (core liquid), and shell liquid was prepared by dissolving a shell material, which comprised 70 parts by mass of gelatin, 20 parts by mass of glycerin and 10 parts by mass of mannitol, in water while heating. Using the seamless minicapsule manufacturing apparatus "Spherex" (a registered trademark of Freund Inc.), multilayer liquid drops were dropped from the multiple nozzle thereof into a hardening liquid to manufacture seamless capsules having a diameter of 6 mm and having 20% of shell ratio (ratio of the mass of shell to the total mass of the seamless capsule).

### Manufacturing conditions are described below.

Hardening Liquid: Medium chain triglycerides (MCT)
Hardening Temperature: 9°C
Shell Liquid Temperature: 60°C
Number of Capsules Produced: eight / second

### Example 2

As shown in Table 1, seamless capsules were manufactured similarly to in Example 1, except that the composition of the shell material was changed to include 50 parts by mass of gelatin, 20 parts by mass of glycerin and 30 parts by mass of mannitol.

### Example 3

As shown in Table 1, seamless capsules were manufactured similarly to Example 1, except that the composition of the shell material was changed to include 30 parts by mass of gelatin, 20 parts by mass of glycerin and 50 parts by mass of mannitol.

### Comparative Example 1

As shown in Table 1, seamless capsules were manufactured similarly to Example 1, except that the composition of the shell material was changed to include 100 parts by mass of gelatin, 40 parts by mass of glycerin, and 50 parts by mass of flour instead of mannitol.

### Comparative Example 2

As shown in Table 1, seamless capsules were manufactured similarly to Example 1, except that the composition of the shell material was changed to include 100 parts by mass of gelatin, 20 parts by mass of glycerin, and 1.8 parts by mass of milk protein instead of mannitol.

### Comparative Example 3

As shown in Table 1, seamless capsules were manufactured similarly to Example 1, except that the composition of the shell material was changed to include 100 parts by mass of gelatin and 20 parts by mass of glycerin, and mannitol was not included.

**Table 1**

| (Parts by mass) | Ex. 1 | Ex. 2 | Ex. 3 | Com. Ex. 1 | Com. Ex. 2 | Com. Ex. 3 |
|---|---|---|---|---|---|---|
| Gelatin | 70 | 50 | 30 | 100 | 100 | 100 |
| Glycerin | 20 | 20 | 20 | 40 | 20 | 20 |
| Flour | - | - | - | 10 | - | - |
| Milk protein | - | - | - | - | 1.8 | - |
| Mannitol | 10 | 30 | 50 | - | - | - |
| Shell ratio | 20 | 20 | 20 | 20 | 20 | 20 |

Each of the capsules manufactured in Examples 1 to 3 and Comparative Examples 1 to 3 were evaluated by following the tests and comparisons of the results were conducted.

### (1) Evaluation of the size of the crystals which are deposited, and dispersion uniformity of the crystal

The size and dispersion uniformity of the crystal comprised in the each capsules of Examples 1 to 3 and Comparative Examples 1 to 3 were evaluated by observing capsule appearance using a microscope.
Example 1: The crystal of deposited mannitol had a uniform size and uniform distribution, and the capsule was lusterless.
Example 2: The crystal of deposited mannitol had a uniform size and uniform distribution, and the capsule was clearly lusterless.
Example 3: The crystal of deposited mannitol has an uniform size and uniform distribution, and the capsule was clearly lusterless.

Comparative Example 1: Although the dispersed flour had a uniform size and uniform distribution, the capsule was not lusterless.
Comparative Example 2: The dispersed milk protein distribution had neither a uniform size nor uniform distribution.
Comparative Example 3: The capsule was transparent and glossy.

### (2) Evaluation of light shielding ability

Light shielding ability of the shells of the capsules was evaluated by conducting visual observation of the capsule appearances of the each of the capsules of Examples 1 to 3 and Comparative Examples 1 to 3.
Example 1: The capsule surface had excellent light shielding ability, since the surface thereof was lusterless and opaque, and diffused reflection of light was exhibited on the surface.
Example 2: The capsule surface had excelled light shielding ability, since the surface thereof was lusterless and opaque, and diffused reflection of the light was exhibited since the surface had many undulations.
Example 3: The capsule surface had excelled light shielding ability, since the surface thereof is lusterless and opaque, and diffused reflection of the light was exhibited since the surface had many undulations.

Comparative Example 1: The capsule surface was glossy and smooth, and therefore light shielding ability was poor.
Comparative Example 2: The capsule surface was glossy and smooth, and therefore light shielding ability was poor.
Comparative Example 3: The capsule surface was highly glossy and transparent, and the capsule surface does not have light shielding ability.

### (3) Sticking Test

In order to observe anti-sticking effect of each of the seamless capsules under the conditions of high temperature and high humidity, 20 of the seamless capsules of Examples 1 to 3 and Comparative Examples 1 to 3 were separately put as a sample into sample bottles made of No. 7 glass. The bottles were maintained for 48 hours in a thermostat at 40°C and 75% RH, while the tops were not put on the bottles. Then, after the temperature in the thermostat became a room temperature, each sample bottle including each sample was put in the states shown in Table 2, and anti-sticking ability was evaluated by counting the number of capsules fallen down from the bottom of the sample bottle. In Table 2, the degree of the impact strength added to the sample bottle increases as it goes from "the sample bottle was made upside down" to "the sample bottle was dropped from a height of 5cm onto a desk". Here, the numbers in Table 2 mean the accumulated number of capsules fallen in the bottles, which accumulated gradually from "the sample bottle was made upside down" to "the sample bottle was dropped from a height of 5cm onto a desk".

**Table 2**

| Numbers | Bottle was made upside down | Bottle was dropped from a height of I cm onto a desk | Bottle was dropped from a height of 3 cm onto a desk | Bottle was dropped from a height of 5 cm onto a desk |
|---|---|---|---|---|
| Ex. 1 | 16 | 20 | - | - |
| Ex. 2 | 20 | - | - | - |
| Ex.3 | 15 | 20 | - | - |
| Com. Ex. 1 | 0 | 4 | 10 | 20 |
| Com. Ex. 2 | 2 | 4 | 10 | 20 |
| Com. Ex. 3 | 0 | 0 | 3 | 7 |

As a result of Table 2, it was recognized that the seamless capsules of Examples 1 to 3 of the present invention can reduce stickiness of the surface of the capsules, and adhesion of the capsules to the vessel was also reduced. Furthermore, among the results, Example 2, wherein 30 parts by mass of mannitol was included, showed a most excellent anti-stickiness effect.

### (4) Sticking test to teeth when mastication was conducted

Each of the capsules of Examples 1 to 3 and Comparative Examples 1 to 3 were masticated by ten persons (panelists), and the degree of stickiness to the tooth was compared between the capsules. The results are shown in Table 3.

**Table 3**

| | Ex. 1 | Ex. 2 | Ex. 3 | Com. Ex. 1 | Com. Ex. 2 | Com. Ex. 3 |
|---|---|---|---|---|---|---|
| Degree of stickiness of capsule | Hard to adhere to the teeth | Did not adhere to the teeth. | Does not adhere to the teeth. | Adhered to the teeth. | Adhered to the teeth. | Adhered to the teeth. |

As a result of Table 3, it was recognized that the seamless capsules of Examples 1 to 3 of the present invention have an effect of preventing stickiness to the teeth at the tine of mastication.

### INDUSTRIAL APPLICABILITY

The seamless capsules of the present invention do not adhere to each other due to the effect of the crystals which are exposed on the surface of the shell, and sufficient anti-stickiness ability regarding the teeth and the like are achieved, and capsules can be taken out easily from a vessel one by one, since lumps of the capsules are not formed even after capsules are packed in a vessel or the like. Furthermore, manufacture of seamless capsules of the present invention is easy since frictionlessness ability is excellent when capsules are formed and capsules substantially do not adhere to each other.

Furthermore, excellent light shielding ability can be obtained due to the presence of crystals which are exposed on the surface of the shell and crystals deposited in the shell. Accordingly, even if a material which does not have optical stability is encapsulated with a shell, it does not need to include colorant for light shielding or a light shielding agent of an inorganic powder in the shell, and therefore, unpreferable influences, which are caused by adding these additives, on desirability and the like does not occur.

## Claims

1. A seamless capsule including a filler material and a shell which encapsulates the filler, wherein
the shell comprises (a) a shell material and (b) a crystallization agent which is at least one or two or more selected from a group consisting of sorbitol, mannitol, xylitol, erythritol, paratinitt, lactitol, maltitol, trehalose, and saccharose; and
the shell includes crystals which are formed by deposition of the crystallization agent when a shell liquid including the shell material and the crystallization agent is hardened; and
the shell is substantially opaque due to the presence of the crystal.

2. The seamless capsule according to claim 1, wherein the shell includes one or two or more plasticizers selected from a group consisting of glycerol, propylene glycol, and polyethylene glycol.

3. The seamless capsule according to claim 1 or 2, wherein the addition amount of the crystallization agent is 10 to 80% by mass based on the total amount of the shell, excluding water.

4. The seamless capsule according to claim 1 or 2, wherein the mass ratio of the shell and the filler material is 5:95 to 70:30.
